# EUROPEAN PATENT APPLICATION

(11) **EP 4 467 534 A1**
(43) Date of publication of application: **27.11.2024**
(21) Application number: 24174434.1
(22) Date of filing: 07.05.2024
(51) Int. Cl.: C07D 233/60, C07B 53/00, A61P 23/00, A61K 31/4164

(54) **METHOD FOR PREPARING TERT-BUTYL (S)-4-(1-(2,3-DIMETHYLPHENYL)ETHYL)-1H-IMIDAZOLE-1-CARBOXYLATE AND SALTS THEREOF AND ITS USE IN A METHOD FOR PREPARING (S)-4-(1-(2,3-DIMETHYLPHENYL)ETHYL)-1H-IMIDAZOLE AND SALTS THEREOF**

(30) Priority: 25.05.2023 EP 23382487
(71) Applicant: Moehs Ibérica, S.L., 08191 Rubí-Barcelona (ES)
(72) Inventor: MIRANDA, Ronnie Andrés, E-08191 Rubí, Barcelona (ES); COROMINAS, Albert, E-08191 Rubí, Barcelona (ES); LLOVERAS, Isabel, E-08191 Rubí, Barcelona (ES); BAYÓN RUEDA, Juan Pablo, E-08820 El Prat de Llobregat, Barcelona (ES)
(74) Representative: ABG Intellectual Property Law, S.L.

(57) **Abstract**

The present invention relates to a method for preparing dexmedetomidine ((S)-4-(1-(2,3-dimethylphenyl)ethyl)-1*H*-imidazole) or a pharmaceutically acceptable salt and/or solvate thereof via the asymmetric hydrogenation of a methylene derivative. The present invention relates also to methods for preparing tert-butyl (S)-4-(1-(2,3-dimethylphenyl)ethyl)-1/7-imidazole-1-carboxylate or a pharmaceutically acceptable salt and/or solvate thereof useful in the preparation of dexmedetomidine or its salts.

## Description

### Field of the Invention

The present invention relates to a method for preparing dexmedetomidine ((S)-4-(1-(2,3-dimethylphenyl)ethyl)-1*H*-imidazole) or a pharmaceutically acceptable salt and/or solvate thereof, and more particularly its hydrochloride salt, via the asymmetric hydrogenation of a methylene derivative. The present invention relates also to methods for preparing *tert*-butyl (S)-4-(1-(2,3-dimethylphenyl)ethyl)-1*H*-imidazole-1-carboxylate or a pharmaceutically acceptable salt and/or solvate thereof useful in the preparation of dexmedetomidine or its salts.

### Background of the Invention

Dexmedetomidine, also called D-medetomidine or (S)-4-(1-(2,3-dimethylphenyl)ethyl)-1*H-*imidazole, has the following formula **(I)**:

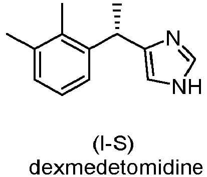

It is the dextrorotatory enantiomer of medetomidine.

The levorotatory enantiomer of medetomidine is called L-medetomidine or levomedetomidine and has the following formula:

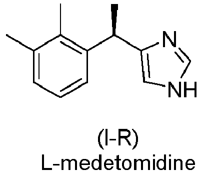

Dexmedetomidine is an α2-agonist sedative drug used in human and veterinary anesthesia. Dexmedetomidine is about twice as powerful as medetomidine, the racemic mixture. It is thus preferable to administer dexmedetomidine instead of medetomidine. Most of the methods reported in the literature to prepare dexmedetomidine involve the preparation of medetomidine, i.e. the racemic mixture, and the separation of the enantiomers via the formation of a diastereoisomer salt. However, in order to obtain dexmedetomidine with a satisfying enantiomeric excess allowing its use as a drug in animal, including man, multiple recrystallisation steps are necessary.

JP 2018-39757 A discloses a method for the preparation of N-protected (S)-4-(1-(2,3-dimethylphenyl)ethyl)-1*H*-imidazoles including but not limited to the preparation of tert-butyl (S)-4-(1-(2,3-dimethylphenyl)ethyl)-1H-imidazole-1-carboxylate involving an asymmetric reduction of a methylene derivative in the presence of certain catalyst and ligands.

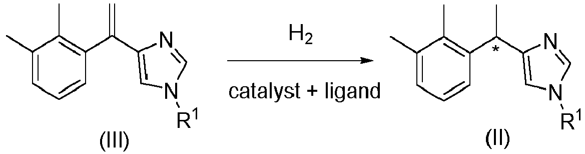

The patent application also describes that the resulting compounds of formula **(II)** can be deprotected to yield a mixture of dexmedetomidine **(I-S)** and L-medetomidine **(I-R)**, designated with formula **(I)**:

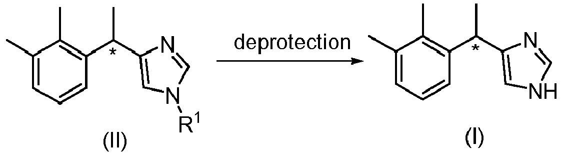

JP 2018-39757 A describes a variety of protecting groups (R¹), catalysts and ligands which can be used in the above process and, depending on the particular choices for these 3 features, conversions ranging from 53.6% and 100% and optical purities ranging from 70.8% and 96.7% are obtained. The overall yield of the enantiomer (product of conversion and optical purity) ranges from 50.5% and 96.7%. The lowest conversion (53.6%) and the lowest overall yield (70.8%) reported in JP 2018-39757 A is obtained when the protecting group (R¹) *tert-*butoxycarbonyl is used. On the other side the greatest overall yield (96.7%) is obtained when (S)-1-[(R)-2-(Diphenylphosphino)ferrocenyl]ethyldi-tert-butylphosphine is used as a ligand.

There is a need for developing a new method for synthesizing a compound of formula **(II),** which can be easily deprotected to yield dexmedetomidine in an efficient manner with a high conversion and a high optical purity.

### Summary of the Invention

The inventors have discovered a new method for a more efficient synthesis of *tert*-butyl (S)-4-(1-(2,3-dimethylphenyl)ethyl)-1*H*-imidazole-1-carboxylate **(IIa)** and its further conversion into dexmedetomidine **(I-S)** or a pharmaceutically acceptable salt and/or solvate thereof as shown in the following scheme: 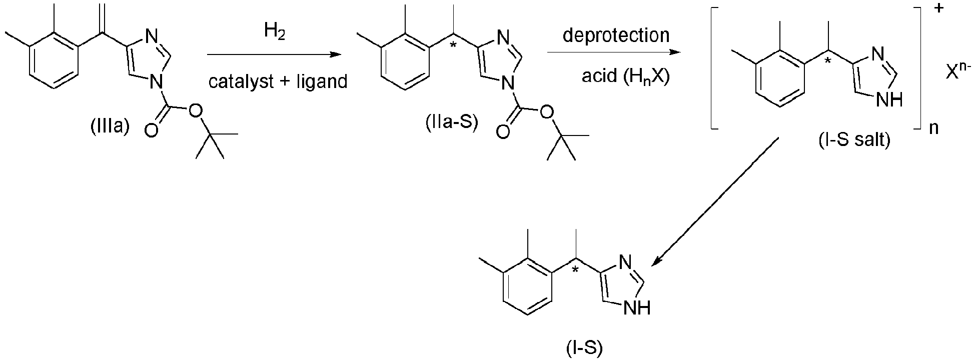 which enables an efficient synthesis of dexmedetomidine or a pharmaceutically acceptable salt and/or solvate thereof, wherein X stands for an anion of a pharmaceutically acceptable acid and n is the charge if the anion.

### Detailed Description of the Invention

In a first aspect, the present invention relates to a process for the preparation of *tert*-butyl (S)-4-(1-(2,3-dimethylphenyl)ethyl)-1*H*-imidazole-1-carboxylate **(IIa-S)** 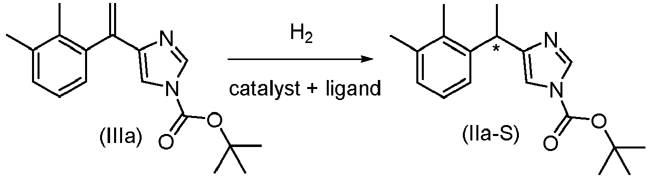 by hydrogenating tert-butyl 4-(1-(2,3-dimethylphenyl)vinyl)-1*H*-imidazole-1-carboxylate using hydrogen gas in a solvent and in the presence of a catalyst selected from the group consisting of rhodium (I) bis(2,5-norbornadiene)tetrafluoroborate **(IVa)** 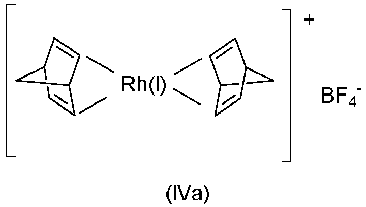 and rhodium (I) bis(2,5-norbornadiene)trifluoromethanesulfonate **(IVb)** 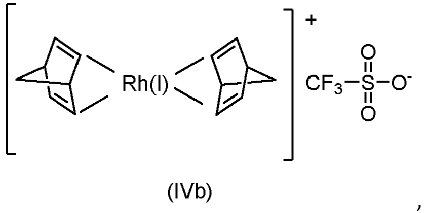 and (S)-1-[(R)-2-[bis[4-(trifluoromethyl)phenyl]phosphino]ferrocenyl]ethyl-di-*tert*-butylphosphine **(V)**

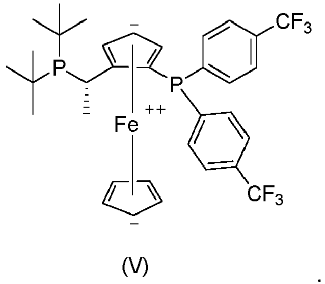

In an embodiment of the first aspect of the present invention the process of the first aspect is carried out in a solvent selected from the group consisting of methanol or mixtures of methanol and dichloromethane.

In another embodiment of the first aspect of the present invention the process is carried out at a temperature between 0 and 30 °C, preferably between 5 and 25°C, more preferably between 15 and 25 °C, more preferably between 18 and 22 °C, more preferably between 5 and 20 °C, most preferably between 19 and 21 °C, preferably 20° C.

In another embodiment of the first aspect of the present invention the process is carried out a pressure of hydrogen gas comprised between 5 and 15 bar, preferably between 8 and 12 bar, more preferably between 9 and 11 bar.

In another embodiment of the first aspect of the present invention the process is carried out using an amount of 0.001 to 0.03, preferably 0.0025 to 0.025, most preferably between 0.005 to 0.02 molar equivalents of a catalyst selected from the group consisting of rhodium (I) bis(2,5-norbornadiene)tetrafluoroborate **(IVa)** and rhodium (I) bis(2,5-norbornadiene)trifluoromethanesulfonate **(IVb).**

In another embodiment of the first aspect of the present invention the process is carried out using an amount of of 0.001 to 0.03, preferably 0.0025 to 0.025, most preferably between 0.005 to 0.02 molar equivalents of (S)-1-[(R)-2-[bis[4-(trifluoromethyl)phenyl]phosphino]ferrocenyl]ethyl-di-*tert*-butylphosphine.

In another embodiment of the first aspect of the present invention the molar ratio of rhodium (I) bis(2,5-norbornadiene)tetrafluoroborate to (S)-1-[(R)-2-[bis[4-(trifluoromethyl)phenyl] phosphino]ferrocenyl]ethyl-di-*tert*-butylphosphine or the molar ratio of rhodium (I) bis(2,5-norbornadiene)trifluoro-methanesulfonate to (S)-1-[(R)-2-[bis[4-(trifluoromethyl)phenyl] phosphino]ferrocenyl]ethyl-di-*tert*-butylphosphine are between 0.8 and 1.2, preferably between 0.9 and 1.1, most preferably 1.

In another embodiment of the first aspect of the present invention, tert-butyl 4-(1-(2,3-dimethylphenyl)vinyl)-1*H*-imidazole-1-carboxylate **(IIIa)** is prepared by reaction of 1-(2,3-dimethylphenyl)-1-(1*H*-imidazol-4-yl)-ethene **(VI)** with a Boc-protecting reagent selected from the groups consisting of di-*tert-*butyl-dicarbonate (Boc₂O), *N-tert-*butoxycarbonyloxy)phthalimide, *tert*-butyl-phenyl carbonate, 1-*tert*-butoxycarbonyl-1,2,4-triazole, 2-(*tert-*butoxycarbonyloxyimino)-2-phenylacetonitrile and 2-(tert-butoxycarbonylthio)-4,6-dimethylpyrimidine, preferably di-*tert*-butyl-dicarbonate (BoczO). In a more particular embodiment the reaction between compound **(VI)** and the Boc-protecting reagent is carried out in the presence of calcium carbonate.

In another embodiment of the first aspect of the present invention, the reaction of 1-(2,3-dimethylphenyl)-1-(1*H*-imidazol-4-yl)-ethene **(VI)** with a Boc-protecting reagent to obtain tert-butyl 4-(1-(2,3-dimethylphenyl)vinyl)-1H-imidazole-1-carboxylate **(IIIa)** and the reaction of hydrogenation of tert-butyl 4-(1-(2,3-dimethylphenyl)vinyl)-1*H*-imidazole-1-carboxylate using hydrogen gas in a solvent and in the presence of a catalyst selected from the group consisting of rhodium (I) bis(2,5-norbornadiene)tetrafluoroborate **(IVa)** and rhodium (I) bis(2,5-norbornadiene)trifluoromethanesulfonate **(IVb),** and (S)-1-[(R)-2-[bis[4-(trifluoromethyl)phenyl] phosphino]ferrocenyl]ethyl-di-tert-butylphosphine **(V)** to obtain tert-butyl (S)-4-(1-(2,3-dimethylphenyl)ethyl)-1*H*-imidazole-1-carboxylate **(IIa-S)** can be performed in the same reactor without isolating tert-butyl 4-(1-(2,3-dimethylphenyl)vinyl)-1*H*-imidazole-1-carboxylate **(IIIa).**

In a second aspect, the present invention relates to a method of preparing dexmedetomidine **(I-S)** or a salt thereof which comprises a) the preparation of *tert*-butyl (S)-4-(1-(2,3-dimethylphenyl)ethyl)-1*H*-imidazole-1-carboxylate **(IIa-S)** as described in the first aspect of the present invention, b) the subsequent deprotection of the imidazole group by cleaving the *tert-*butoxycarbonyl group using an acid. The resulting dexmedetomidine salt may be easily converted to dexmedetomidine using a base. If desired dexmetomidine can be then converted into a pharmaceutically acceptable salt by treatment with a pharmaceutically acceptable acid

In an embodiment of the second aspect of the invention the acid used in step b) is selected from the group consisting of hydrochloric acid and trifluoroacetic acid, in particular hydrochloric acid.

In a third aspect, the present invention relates to a method of preparing tert-butyl 4-(1-(2,3-dimethylphenyl)vinyl)-1H-imidazole-1-carboxylate **(IIIa)** by reacting 1-(2,3-dimethylphenyl)-1-(1H-imidazol-4-yl)-ethene **(VI)** with a Boc-protecting reagent. Examples of Boc-protecting reagents are di-*tert*-butyl-dicarbonate (Boc₂O), *N-tert*-butoxycarbonyloxy)phthalimide, *tert-*butyl-phenyl carbonate, 1-*tert*-butoxycarbonyl-1,2,4-triazole, 2-(*tert*-butoxycarbonyloxyimino)-2-phenylacetonitrile and 2-(tert-Butoxycarbonylthio)-4,6-dimethylpyrimidine, preferably di-*tert-*butyl-dicarbonate (Boc₂O). The particular conditions for carrying out the protection using the above-mentioned reagents are well known to the expert in the field.

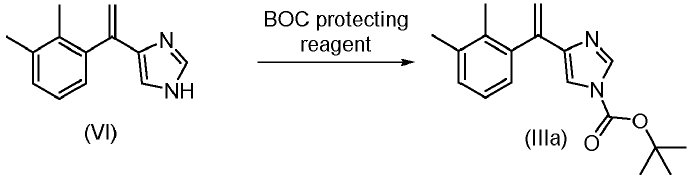

Dexmedetomidine salts are preferably addition salts with pharmaceutically acceptable acids that are typically formed by reacting a compound of formula **(I-S)** with an equivalent amount or with an excess of acid. The inorganic acids commonly used for forming such salts include hydrochloric acid, hydrobromic acid, hydroiodic acid, sulfuric acid, phosphoric acid, and the like. The organic acids commonly used for forming such salts include p-toluenesulfonic acid, methanesulfonic acid, oxalic acid, p-bromophenylsulfonic acid, carbonic acid, succinic acid, citric acid, benzoic acid, acetic acid, and the like. Examples of such pharmaceutically acceptable salts are sulfate, pyrosulfate, bisulfate, sulfite, bisulfite, phosphate, monohydrogen phosphate, dihydrogen phosphate, metaphosphate, pyrophosphate, chloride, bromide, iodide, propionate acetate, decanoate, caprylate, acrylate, formate, heptanoate, propiolate, oxalate, malonate, succinate, hemisuccinate, suberate, sebacate, fumarate, maleate, butyne-1,4-dioate, 1-hexyne, 6-dioate, benzoate, chlorobenzoate, methylbenzoate, dinitrobenzoate, hydroxybenzoate, benzoate, benzoate, benzoate, benzoate, metobenzoate, hydroxybenoate, hydroxybenzoate, hydroxybenzoate, sulfonate, xylenesulfonate, phenylacetate, phenylpropionate, phenylbutyrate, citrate, lactate, p-hydroxybutyrate, glycolate, tartrate, methanesulfonate, propanesulfonate, naphthalene-1-sulfonate, naphthalene-2-sulfonate, mandelate. The preferred pharmaceutically acceptable salts are those formed with hydrochloric acid and succinic acid, preferably hydrochloric acid.

### Examples

### General Protocols

The conversion and enantiomeric purity of the products obtained in examples 5 to 9 have been analyzed by means of the ultra high-resolution liquid chromatography (UHPLC) technique in an Acquity model, Waters brand apparatus, provided with a variable wavelength UV detector, a mass detector, and a thermostatic oven for the column. Chiralpak AGP, 150 x 4.0 mm x 5µm column and mobile phases A (17.3 mM of sodium phosphate dibasic dihydrate and 12.2 mM Sodium phosphate monobasic monohydrate, pH 7) y B (acetonitrile) were used with the following conditions of analysis:
Flow: 1.0 mL/min
T^{a} column: 25 °C
Wavelenght: 220 nm
Injection volume: 20 µL
Mobile phase: Phase A/Phase B (85:15)

The purity of products obtained in reference examples 1 ,3 and 4 have been analyzed by means of the ultra high-resolution liquid chromatography (UHPLC) technique in an Acquity model, Waters brand apparatus, provided with a variable wavelength UV detector, a mass detector, and a thermostatic oven for the column. Acquity HSS T3, 100 x 3.0 mm x 1.8 µm column and mobile phases A (dissolve 0.95 g of ammonium acetate in 220 ml of water, adjust to pH 5.2 with acetic acid diluted solution and dilute to 250 ml with water), B (acetonitrile) and C (water) were used with the following conditions of analysis:
Flow: 0.5 mL/min
T^{a} column: 40 °C
Wavelenght: 220 nm
Injection volume: 2 µL
Mobile phase: Phase B/Phase C (50:50)
Gradient:

| t (min) | %A | %B | %C |
|---|---|---|---|
| 0 | 5 | 10 | 85 |
| 0.5 | 5 | 10 | 85 |
| 10 | 5 | 90 | 5 |
| 11.5 | 5 | 90 | 5 |
| 12 | 5 | 10 | 85 |
| 15 | 5 | 10 | 85 |

### Reference example 1: Synthesis of (2,3-dimethylphenyl)-(1H-imidazol-4-yl)-methanone

37.6 g (1.547 mol) of magnesium were mixed with 1100 ml of dry THF. The suspension containing magnesium is inertized under N₂ atmosphere and 30 mL (0.03 mol) of a 1M solution in THF of diisobutylaluminium hydride (DIBALH) were slowly added at a temperature of about 20 °C. Then, the obtained suspension was warmed up to a temperature of between 35-40°C and a previously prepared solution of 150 ml (1.107 mol) of 2,3-dimethylbromobenzene and 250 ml of dry THF were slowly added at the said temperature. The obtained mixture was warmed up at reflux temperature and maintained for 1 hour.

Once the maintenance was finished, the mixture comprising the obtained 2,3-dimethylphenylmagnesium bromide was cooled to a temperature of between 20-25°C and maintained under N₂ atmosphere.

40 g (0.430 mol) of 4-cyanoimidazole and 200 ml of THF were mixed at a temperature of between 0 and 5 °C. The mixture was inertized with N₂ atmosphere. The previously obtained suspension comprising 2,3-dimethylphenylmagnesium bromide was slowly transferred to the suspension of 4-cyanoimidazole maintaining the temperature of between 0 and 5 °C. The mixture was stirred for 3h at this temperature.

Once the maintenance was finished, 250 ml (1.778 mol) of a 30% by weight aqueous solution of H₂SO₄ and 250 ml of H₂O was slowly added to the reaction mixture maintaining the temperature below the temperature of about 30 °C. The resulting suspension is filtered and washed with 6 fractions of 500 ml of THF. Then, 3500 mL of heptane and 2500 mL of water were added. The organic phase was separated and the aqueous phase was washed with 3500 ml of heptane. 4000 ml of water were added to the resulting aqueous phase. The obtained aqueous solution was cooled to a temperature of between 0 and 5 °C and an aqueous solution of NH3 was added to pH 9.8- 11.0 at a temperature below of 10 °C. The resulting suspension was cooled to a temperature of between 0 and 5 °C and maintained for 2 hours at the said temperature. The resulting suspension was centrifugated to afford a solid that was washed with four fractions of 5000 ml of water and two fractions of 3000 ml of heptane and dried with vacuum at a temperature of about 40 °C to obtain 77.49 g (90.0% yield, 99.78% UHPLC purity) of an off-white solid corresponding to (2,3-dimethylphenyl)-(1H-imidazol-4-yl)-methanone.

### Reference example 2: Synthesis of 1-(2,3-dimethylphenyl)-1-(1H-imidazol-4-yl)-ethan-1-ol

75.0 g (0.375 mol) of (2,3-dimethylphenyl)-(1H-imidazol-4-yl)-methanone and 600 ml of THF were mixed to obtain a suspension, which is inertized with N₂ atmosphere. 370 ml (1.11 mol) of a 3M solution in THF of methylmagnesium chloride were added at a temperature of about 20 °C. The obtained suspension was warmed up to a temperature between 60 and 65 °C and maintained under stirring at said temperature for 3 hours.

Once the maintenance was finished, the mixture was cooled down to a temperature between 0 and 10 °C. A solution of 111.6 g of ammonium chloride in 375 ml of water was slowly added at a temperature below 30 °C. Then 300 ml of water were added and a solution of HCI 37% in weight was added until pH of about 7. 150 ml of ethyl acetate were added and the phases were separated. 150 ml of ethyl acetate were added and the phases were separated again. The combined organic phases were washed with the addition of a solution of 107.25 g of NaCl in 386 ml of water. The solvent of the obtained organic phase was eliminated by means of distillation under vacuum to obtain a residue comprising 1-(2,3-dimethylphenyl)-1-(1H-imidazol-4-yl)-ethan-1-ol, which was used in the next reaction without additional treatment.

### Reference example 3: Synthesis of 1-(2,3-dimethylphenyl)-1-(1H-imidazol-4-yl)-ethene

412.5 ml of toluene and 7.12 g (0.0374 mol) of para-toluenesulfonic acid monohydrate were added to the residue previously obtained in reference example 2. The mixture was warmed up to a temperature of between 105 and 110 °C and maintained under stirring for 2 hours.

Once the maintenance was finished, the mixture was cooled down to a temperature of about 50 °C and a solution of 15.73 g of NaHCOs in 210 ml of water was slowly added. The mixture was cooled down to a temperature of between 35 and 40 °C and let to crystallize. Once the mixture started to crystallize, it was cooled down to a temperature of about 0 °C and maintained for 2 hours. The resulting suspension was filtered to afford a solid that was washed with two fractions of 82.5 ml of water and two fractions of 82.5 ml of heptane and dried with vacuum at a temperature of about 40 °C to obtain 70.9 g (95.4% yield, 99.88% UHPLC purity) of a white solid corresponding to 1-(2,3-dimethylphenyl)-1-(1H-imidazol-4-yl)-ethene.

### Reference example 4: Synthesis of tert-butyl-4-[1-(2,3-dimethylphenyl)vinyl]-1H-imidazole-1-carboxylate (IIIa)

10 g of 1-(2,3-dimethylphenyl)-1-(1H-imidazol-4-yl)-ethene **(VI)** (50.5 mmol) are suspended in 70 ml of dichloromethane at a temperature of about 20 °C. 10.46 g of K₂CO₃ (75.7 mmol) are added, the obtained mixture is warmed up to reflux and 11.56 g of BoczO (52.9 mmol) are slowly added. The mixture is stirred at reflux temperature for 3 hours.

Once the maintenance is finished, the mixture is cooled down to a temperature of about 20 °C and filtered. The solvent of the obtained solution is eliminated by means of distillation under vacuum. 50 mL of n-heptane are added to the residue to obtain a fluid mass under stirring. The mixture is cooled to a temperature between 0 and 5 °C and maintained at this temperature for 2 hours. The resulting suspension was filtered to afford a solid that was washed two times with 5 mL each time of n-heptane and dried with vacuum at a temperature of about 50 °C to obtain 12.8 g (85.0% yield, 99.85% UHPLC purity) of a white solid corresponding to tert-butyl-4-[1-(2,3-dimethylphenyl)vinyl]-1*H*-imidazole-1-carboxylate **(IIIa).**

### Example 5: Synthesis of tert-butyl-4-[(1S)-1-(2,3-dimethylphenyl)ethyl]-1H-imidazole-1-carboxylate (IIa-S)

8.96 g of tert-butyl-4-[1-(2,3-dimethylphenyl)vinyl]-1H-imidazole-1-carboxylate, 0.02 molar equivalents of bis(norbornadiene)rhodium(I) tetrafluoroborate and 0.02 molar equivalents of (S)-1-[(R)-2-[bis[4-(trifluoromethyl)phenyl]phosphino]ferrocenyl]ethyldi-tert-butylphosphine are dissolved in 14 volumes of methanol. Three successive inertization sequences were performed with N₂ and vacuum and, lastly, the inner pressure of the flask was adjusted to about 10 bars with H₂ atmosphere. The resulting mixture was stirred to a temperature of about 20 °C for 24 hours.

Once the maintenance was finished, a conversion of 100 % and an optical purity of 97.5% by UHPLC for tert-butyl-4-[(1S)-1-(2,3-dimethylphenyl)ethyl]-1H-imidazole-3-carboxylate **(IIa-S)** were observed.

### Example 6: Synthesis of tert-butyl-4-[(1S)-1-(2,3-dimethylphenyl)ethyl]-1H-imidazole-1-carboxylate (IIa-S)

8.00 g of tert-butyl-4-[1-(2,3-dimethylphenyl)vinyl]-1H-imidazole-1-carboxylate, 0.03 molar equivalents of bis(norbornadiene)rhodium(I) tetrafluoroborate and 0.03 molar equivalents of (S)-1-[(R)-2-[bis[4-(trifluoromethyl)phenyl]phosphino]ferrocenyl]ethyldi-tert-butylphosphine are dissolved in a mixture of 14 volumes of methanol and 4.6 volumes of dichloromethane. Three successive inertization sequences were performed with N₂ and vacuum and, lastly, the inner pressure of the flask was adjusted to about 10 bars with H₂ atmosphere. The resulting mixture was stirred to a temperature of about 10 °C for 24 hours.

Once the maintenance was finished, a conversion of 99.55% and an optical purity of 97.66% by UHPLC for tert-butyl-4-[(1S)-1-(2,3-dimethylphenyl)ethyl]-1H-imidazole-3-carboxylate **(IIa-S)** were observed.

### Example 7: Synthesis of tert-butyl-4-[(1S)-1-(2,3-dimethylphenyl)ethyl]-1H-imidazole-1-carboxylate (IIa-S)

3.50 g of tert-butyl-4-[1-(2,3-dimethylphenyl)vinyl]-1H-imidazole-1-carboxylate, 0.03 molar equivalents of bis(norbornadiene)rhodium(I) tetrafluoroborate and 0.03 molar equivalents of (S)-1-[(R)-2-[bis[4-(trifluoromethyl)phenyl]phosphino]ferrocenyl]ethyldi-tert-butylphosphine are dissolved in a mixture of 14 volumes of methanol and 4.6 volumes of dichloromethane. Three successive inertization sequences were performed with N₂ and vacuum and, lastly, the inner pressure of the flask was adjusted to about 10 bars with H₂ atmosphere. The resulting mixture was stirred to a temperature of about 20 °C for 24 hours.

Once the maintenance was finished, a conversion of 99.63% and an optical purity of 97.51% by UHPLC for tert-butyl-4-[(1S)-1-(2,3-dimethylphenyl)ethyl]-1H-imidazole-3-carboxylate (lla-S) were observed.

### Example 8: Synthesis of tert-butyl-4-[(1S)-1-(2,3-dimethylphenyl)ethyl]-1H-imidazole-1-carboxylate (IIa-S)

7.52 g of tert-butyl-4-[1-(2,3-dimethylphenyl)vinyl]-1H-imidazole-1-carboxylate, 0.005 molar equivalents of bis(norbornadiene)rhodium(I) trifluoromethanesulfonate and 0.005 molar equivalents of (S)-1-[(R)-2-[bis[4-(trifluoromethyl)phenyl]phosphino]ferrocenyl]ethyldi-tert-butylphosphine are dissolved in a mixture of 4.65 volumes of methanol and 2.33 volumes of dichloromethane.Three successive inertization sequences were performed with N₂ and vacuum and, lastly, the inner pressure of the flask was adjusted to about 10 bars with H₂ atmosphere. The resulting mixture was stirred to a temperature of about 5 °C for 24 hours.

Once the maintenance was finished, a conversion of 99.78% and an optical purity of 98.26% by UHPLC for tert-butyl-4-[(1S)-1-(2,3-dimethylphenyl)ethyl]-1H-imidazole-3-carboxylate (lla-S) were observed.

### Example 9: Synthesis of tert-butyl-4-[(1S)-1-(2,3-dimethylphenyl)ethyl]-1H-imidazole-1-carboxylate (IIa-S)

7.52 g of tert-butyl-4-[1-(2,3-dimethylphenyl)vinyl]-1H-imidazole-1-carboxylate, 0.02 molar equivalents of bis(norbornadiene)rhodium(I) trifluoromethanesulfonate and 0.02 molar equivalents of (S)-1-[(R)-2-[bis[4-(trifluoromethyl)phenyl]phosphino]ferrocenyl]ethyldi-tert-butylphosphine are dissolved in a mixture of 14 volumes of methanol and 4.6 volumes of dichloromethane. Three successive inertization sequences were performed with N₂ and vacuum and, lastly, the inner pressure of the flask was adjusted to about 10 bars with H₂ atmosphere. The resulting mixture was stirred to a temperature of about 5 °C for 24 hours.

Once the maintenance was finished, a conversion of 99.53% and an optical purity of 97.33% by UHPLC for tert-butyl-4-[(1S)-1-(2,3-dimethylphenyl)ethyl]-1H-imidazole-3-carboxylate (lla-S) were observed.

### Comparative example 10: Synthesis of tert-butyl-4-[(1S)-1-(2,3-dimethylphenyl)ethyl]-1H-imidazole-1-carboxylate (IIa-S)

5.00 g of tert-butyl-4-[1-(2,3-dimethylphenyl)vinyl]-1H-imidazole-1-carboxylate, 0.02 molar equivalents of bis(norbornadiene)rhodium(I) tetrafluoroborate and 0.02 molar equivalents of (S)-1-[(R)-2-(Diphenylphosphino)ferrocenyl]ethyldi-tert-butylphosphine are dissolved in 14 volumes of methanol. Three successive inertization sequences were performed with N₂ and vacuum and, lastly, the inner pressure of the flask was adjusted to about 10 bars with H₂ atmosphere. The resulting mixture was stirred to a temperature of about 20 °C for 24 hours.

Once the maintenance was finished, a conversion of 58.90 % and an optical purity of 94.58 % by UHPLC for tert-butyl-4-[(1S)-1-(2,3-dimethylphenyl)ethyl]-1H-imidazole-3-carboxylate **(IIa-S)** were observed.

The results obtained in Example 5, 6, 7, 8, 9 and Comparative example 10 are summarized in table 1 below:

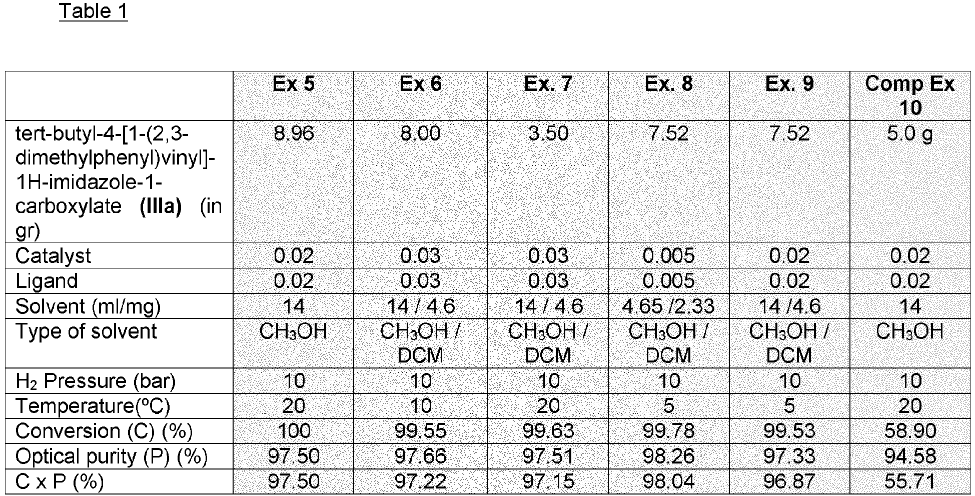

The results summarized on table 1 clearly show that the process of the present invention allows to obtain tert-butyl-4-[(1S)-1-(2,3-dimethylphenyl)ethyl]-1H-imidazole-1-carboxylate **(IIa-S)** with very high optical purity. Actually, the optical purities obtained are higher than those reported in JP 2018-39757 using any combination of protecting group, catalyst and ligand (which range from 70.8% to 96.7%).

### Example 11: Synthesis of 4-[(1S)-1-(2,3-dimethylphenyl)ethyl]-1H-imidazole (dexmedetomidine) (I-S)

*tert*-butyl-4-[(1S)-1-(2,3-dimethylphenyl)ethyl]-1H-imidazole-3-carboxylate (IIa-S) was prepared as described in Example 5 above. After the reacting mixture had been stirred to a temperature of about 20°C for 24 hours as explained in example 5, the solvent was eliminated by means of distillation under vacuum. 7.2 ml of water and 5.2 ml (62.7 mmol) of a 37% by weight aqueous solution of HCI were added to the residue. The mixture obtained was warmed up to a temperature of about 50 °C, stirred for 1 hour to this temperature and cooled down to a temperature of about 20 °C. The resulting mixture contained dexmedetomidine in the form of its hydrochloride **(I-S HCl).** Then, 6.4 ml (62.4 mmol) of a 30% by weight aqueous solution of NaOH were slowly added to the mixture and then 19.7 ml of acetone. The mixture was warmed up to reflux temperature and stirred for 15 minutes. The obtained solution was slowly cooled down to a temperature of between 0 and 5 °C and maintained at this temperature for 1 hour. The resulting suspension was filtered to afford a solid that was washed with 18 mL of a mixture 1:1 of acetone/water and then with 9 ml of water and dried with vacuum at a temperature of about 50 °C to obtain 4.56 g (75.8% yield, 99.86% UHPLC optical purity) of a white solid corresponding 4-[(1S)-1-(2,3-dimethylphenyl)ethyl]-1H-imidazole (dexmedetomidine) **(I-S)**.

### Example 12: Synthesis of hydrochloride salt of 4-[(1S)-1-(2,3-dimethylphenyl)ethyl]-1H-imidazole (hydrochloride salt of dexmedetomidine) (I-S HCl)

50 g (0.250 mol) of 4-[(1S)-1-(2,3-dimethylphenyl)ethyl]-1H-imidazole (dexmedetomidine) **(I-S)** obtained by means of procedure of example 11 and 263.5 ml of a 1.2 M solution of hydrogen chloride in ethanol were mixed to obtain a solution, which was filtered throw 1 µm filter and the filter was washed with 3 fractions of 75 ml each of ethanol. The solvent of the obtained solution was eliminated by means of distillation under vacuum to obtain a residue. 100 ml of acetone were added to the residue and the solvent was eliminated by means of distillation under vacuum to obtain a residue. 100 ml of acetone were added to the residue and the solvent was eliminated by means of distillation under vacuum to obtain a residue. 150 ml of acetone was added to the residue and the mixture was warmed up to a temperature of between 50 and 55 °C and stirred at said temperature for 2 hours. The mixture was cooled down to a temperature of between 0 and 5 °C and maintained under stirring for 2 hours. The resulting suspension was filtered to afford a solid that was washed with 25 mL of acetone and dried with vacuum at a temperature of about 40 °C to obtain 54.75 g (92.5% yield, 99.95% HPLC chemical purity, 99.90% UHPLC optical purity) of a white solid corresponding to hydrochloride salt of 4-[(1S)-1-(2,3-dimethylphenyl)ethyl]-1 H-imidazole (hydrochloride salt of dexmedetomidine) **(I-S HCl)**.

## Claims

1. A process for the preparation of tert-butyl (S)-4-(1-(2,3-dimethylphenyl)ethyl)-1*H*-imidazole-1-carboxylate **(IIa-S)** 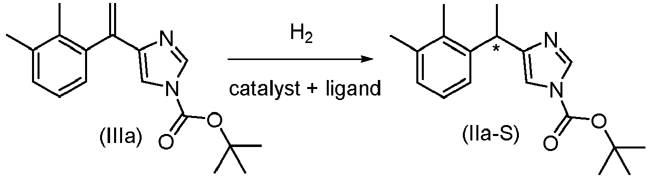 by hydrogenating tert-butyl 4-(1-(2,3-dimethylphenyl)vinyl)-1*H*-imidazole-1-carboxylate **(IIIa)** using hydrogen gas in a solvent in the presence of a catalyst selected from the group consisting of rhodium (I) bis(2,5-norbornadiene)tetrafluoroborate **(IVa)** and 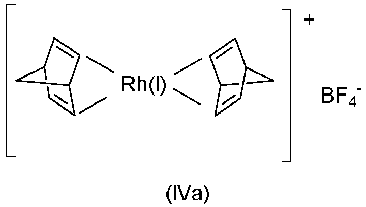 and rhodium (I) bis(2,5-norbornadiene)trifluoromethanesulfonate (IVb) 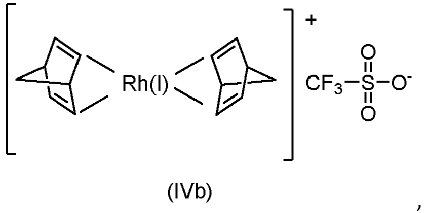 and (S)-1-[(R)-2-[bis[4-(trifluoromethyl)phenyl]phosphino]ferrocenyl]ethyl-di-*tert*-butylphosphine **(V)**

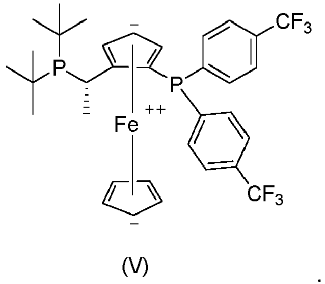

2. A process according to claim 1 **characterized in that** the catalyst is rhodium (I) bis(2,5-norbornadiene)tetrafluoroborate **(IVa).**

3. A process according to claim 1 **characterized in that** the catalyst is rhodium (I) bis(2,5-norbornadiene)trifluoromethanesulfonate **(IVb).**

4. A process according to any one of claims 1 to 3 **characterized in that** the solvent is selected from the group consisting of methanol or mixtures of methanol and dichloromethane.

5. A process according to any one of claims 1 or 4 **characterized in that** it is carried out at a temperature between 0 and 30 °C, preferably between 5 and 25°C, , more preferably between 5 and 20 °C.

6. A process according to any one of claims 1 to 5 **characterized in that** it is carried out at a pressure of hydrogen gas comprised between 5 and 15 bar, preferably between 8 and 12 bar, more preferably between 9 and 11 bar.

7. A process according to any one of claims 1 to 6 **characterized in that** it is carried out using an amount of 0.001 to 0.03, preferably 0.0025 to 0.025, preferably, most preferably between 0.005 to 0.02 molar equivalents of the catalyst **(IVa)** or **(IVb).**

8. A process according to any one of claims 1 to 7 **characterized in that** it is carried out using an amount of of 0.001 to 0.03, preferably 0.0025 to 0.025, most preferably between 0.005 to 0.02 molar equivalents of (S)-1-[(R)-2-[bis[4-(trifluoromethyl)phenyl]phosphino]-ferrocenyl]ethyl-di-tert-butylphosphine.

9. A process according to any one of claims 1 to 8 **characterized in that** it is carried out using a molar ratio of rhodium (I) bis(2,5-norbornadiene)tetrafluoroborate to (S)-1-[(R)-2-[bis[4-(trifluoromethyl)phenyl]phosphino]ferrocenyl]ethyl-di-tert-butylphosphine or a molar ratio of rhodium (I) bis(2,5-norbornadiene)trifluoromethanesulfonate to (S)-1-[(R)-2-[bis[4-(trifluoromethyl)phenyl]phosphino]ferrocenyl]ethyl-di-tert-butylphosphine between 0.8 and 1.2, preferably between 0.9 and 1.1, most preferably 1.

10. A process according to any one of claims 1 to 9 wherein tert-butyl 4-(1-(2,3-dimethylphenyl)vinyl)-1*H*-imidazole-1-carboxylate **(IIIa)** is prepared by reaction of 1-(2,3-dimethylphenyl)-1-(1H-imidazol-4-yl)-ethene **(VI)** with a Boc-protecting reagent selected from the groups consisting of di-*tert*-butyl-dicarbonate (Boc₂O), *N*-*tert-*butoxycarbonyloxy)phthalimide, *tert-*butyl-phenyl carbonate, 1-*tert*-butoxycarbonyl-1,2,4-triazole, 2-(*tert-*butoxycarbonyloxyimino)-2-phenylacetonitrile and 2-(tert-Butoxycarbonylthio)-4,6-dimethylpyrimidine, preferably di-*tert*-butyl-dicarbonate (Boc₂O)

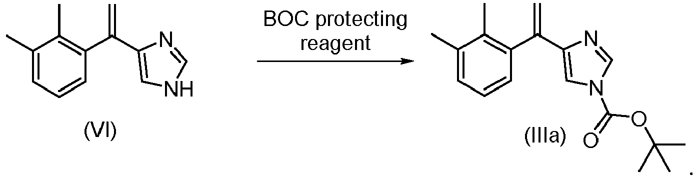

11. - A process according to any one of claims 1 to 10 wherein the reaction between compound **(VI)** and the Boc-protecting reagent is carried out in the presence of calcium carbonate.

12. A process according to claim 11 wherein the Boc-protecting reagent is di-*tert*-butyl-dicarbonate.

13. A process for the preparation of dexmedetomidine **(I-S)** or a salt thereof which comprises:
a) the preparation of *tert*-butyl (S)-4-(1-(2,3-dimethylphenyl)ethyl)-1*H*-imidazole-1-carboxylate **(IIa-S)** according to any one of claims 1 to 12 and
b) the subsequent deprotection of the imidazole group of compound **(IIa-S)** by cleaving the tert-butoxycarbonyl group using an acid, and
c) optionally, when it is desired to obtain dexmedetomidine **(I-S)** in the form of the free base, the neutralization of the product resulting from step b) with a base.

14. The process according to claim 13 wherein the acid is selected from the group consisting of hydrochloric acid and trifluoroacetic acid, in particular hydrochloric acid.
